# EUROPÄISCHE PATENTSCHRIFT

(11) **EP 1 918 092 B1**
(45) Veröffentlichungstag und Bekanntmachung des Hinweises auf die Patenterteilung: **06.02.2013**
(21) Anmeldenummer: 06022799.8
(22) Anmeldetag: 02.11.2006
(51) Int. Cl.: B32B 3/22, B32B 5/26

(54) **Verbundstoffbahn und Verfahren zur Herstellung einer Verbundstoffbahn**
Composite fabric and method for making the same
Bande textile composite, ainsi que son procédé de fabrication

(43) Veröffentlichungstag der Anmeldung: 07.05.2008
(73) Patentinhaber: Mondi Consumer Packaging Technologies GmbH, 48599 Gronau (DE)
(72) Erfinder: Baldauf, Georg, 48366, Laer (DE); Willing, Christoph, 48691, Vreden (DE); Schönbeck, Marcus, 33775, Versmold (DE)
(74) Vertreter: Albrecht, Rainer Harald

(56) Entgegenhaltungen:
- EP-A- 1 736 306
- EP-A1- 1 331 090
- EP-A1- 1 342 562
- EP-A1- 1 686 209
- WO-A-98/16380
- US-A1- 2003 194 936
- US-B1- 6 461 715

## Beschreibung

Die Erfindung betrifft eine Verbundstoffbahn mit zwei außenliegenden Vliesstoffbahnen und dazwischen abschnittsweise angeordneten Folienstreifen einer elastischen Folie, wobei die Vliesstoffbahnen in den Bereichen zwischen den Folienstreifen miteinander verbunden sind.

Die Verbundstoffbahn kann beispielsweise zur Herstellung elastischer Verschlussstreifen für Babywindeln oder Erwachsenenwindeln verwendet werden, welche auch die Form von Windelohren aufweisen können und jeweils einen elastischen mittleren Bereich sowie daran beidseitig anschließende weniger elastische Endabschnitte besitzen. Die nicht bzw. weniger elastischen Endbereiche werden genutzt, um Verschlusselemente, z. B. Hakentapes zu befestigen und den Verschlussstreifen am Windelchassis anzubringen. Zur kostengünstigen Fertigung wird eine große Zahl von elastischen Folienstreifen mit Abstand zueinander zwischen breiten Bahnen aus Vliesstoff einkaschiert. Aus der resultierenden Verbundstoffbahn werden dann die für die Windelherstellung benötigten Verschlussstreifen abgetrennt.

Eine Verbundstoffbahn mit den eingangs beschriebenen Merkmalen ist aus der Druckschrift EP 1 686 209 A1 bekannt. Die Folienstreifen weisen hier einen elastischen, klebrigen Kern auf, welcher ein- bzw. beidseitig mit einer dünnen Nonwoven-Schicht abgedeckt ist, um trotz der Klebrigkeit des Kernes eine Verarbeitung der Folienstreifen zu ermöglichen. Um die gewünschte Elastizität der Verbundstoffbahnen zu gewährleisten, werden diese einem Ringrollprozess unterworfen. Hierdurch bilden sich in den gedehnten und nicht elastisch rückstellbaren Vliesstoffbahnen Verwerfungen, welche bei einem Dehnvorgang glatt gezogen werden.

Eine einfache Möglichkeit zur Herstellung einer Verbundstoffbahn mit den gewünschten Eigenschaften besteht darin, zwei Vliesstoffstreifen durch eine elastische Folie miteinander zu verbinden. Der asymmetrische Aufbau eines derartigen Laminates neigt jedoch zum Abreißen im Bereich der im Allgemeinen durch Klebstoff hergestellten Verbindung zwischen Folie und Vliesstoffstreifen. Ferner ist nachteilig, dass die elastische Folie in diesem Fall freiliegt und damit die Verbundstoffbahn in diesem Bereich nicht den angenehmen Textilcharakter der restlichen Windeloberfläche besitzt.

Der Erfindung liegt die Aufgabe zugrunde, eine Verbundstoffbahn zur Herstellung von Verschlusselementen für Windeln, insbesondere Erwachsenenwindeln, anzugeben, welche einfach herstellbar ist und gleichzeitig eine ausreichend hohe Stabilität besitzt.

Ausgehend von einer Verbundstoffbahn mit den eingangs beschriebenen Merkmalen wird die Aufgabe erfindungsgemäß dadurch gelöst, dass die Folienstreifen lediglich an jeweils beiden Längsrändern sowohl ober- als auch unterseitig mit den Innenflächen der Vliesstoffbahnen verbunden sind und dass die Vliesstoffbahnen in den die Folienstreifen überdeckenden Bereichen in Bahnrichtung verlaufende Schwächung aufweisen. Die Schwächung kann als Perforation oder alternativ auch als Schlitzung oder Anritzung ausgebildet sein. Durch die beidseitige Verbindung des Folienstreifens mit den außenliegenden Vliesstoffbahnen wird ein sehr stabiler Verbund erzielt, der den Belastungen eines Windelverschlusselementes ohne weiteres standhält. Streckt man die erfindungsgemäße Verbundstoffbahn in Querrichtung, so zerreißt die Schwächung und der elastische Folienstreifen wird freigelegt. Somit lässt sich die Elastizität des Folienstreifens voll nutzen. Die erstmalige Streckung des mit der Schwächung versehenen Windelverschlusselementes erfolgt in der Regel durch den Windelanleger, bei Erwachsenenwindeln entsprechend ggf. durch den Windelträger selbst.

Vorzugsweise weisen die Vliesstoffbahnen pro Folienstreifen jeweils eine Schwächung auf und die Schwächungen verlaufen jeweils symmetrisch zum Folienstreifen. Zweckmäßigerweise erstrecken sich die Schwächungen über die gesamte Länge der Folienstreifen. Zur Herstellung nicht elastischer Abschnitte können die Vliesstoffbahnen in den Bereichen zwischen den Folienstreifen miteinander verklebt sein. Zweckmäßigerweise sind auch die Randbereiche der Folienstreifen mit den Innenflächen der Vliesstoffbahnen verklebt. Als Klebstoffe können in beiden Fällen Hotmeltkleber eingesetzt werden, die mittels eines Kaschiervorganges den Verbund herstellen.

Um auch nach dem Freilegen des elastischen Folienstreifens einen vollflächigen Textilcharakter des Windelverschlusselementes beizubehalten, können die Folienstreifen jeweils aus einer Elastomerschicht und zumindest einer auf die Elastomerschicht aufgebrachten Außenschicht aus Vliesstoff bestehen. Da die Elastomerschicht häufig klebrig ist, kann es zweckmäßig sein, diese beidseitig mit Außenschichten aus Vliesstoff zu versehen. Die Vliesstoffaußenschichten können eine wesentlich geringere Dicke als die Vliesstoffbahnen aufweisen, um die ihr zugedachten Funktionen - also Textilcharakter einerseits und Abdeckung der klebrigen Elastomerschicht andererseits - zu erfüllen.

Gegenstand der Erfindung ist ferner ein Verfahren zur Herstellung einer Verbundstoffbahn gemäß Anspruch 8. Zweckmäßige Ausgestaltungen dieses Verfahrens sind in den Ansprüchen 9 und 10 beschrieben.

Im Folgenden wird die Erfindung anhand einer lediglich ein Ausführungsbeispiel darstellenden Zeichnung ausführlich erläutert. Es zeigen schematisch:
- **Fig. 1**: ein aus einer erfindungsgemäßen Verbundstoffbahn hergestelltes Windelverschlusselement in Querschnittsdarstellung,
- **Fig. 2**: die Draufsicht X in Fig. 1 und
- **Fig. 3**: ein erfindungsgemäßes Verfahren zur Herstellung einer Verbundstoffbahn.

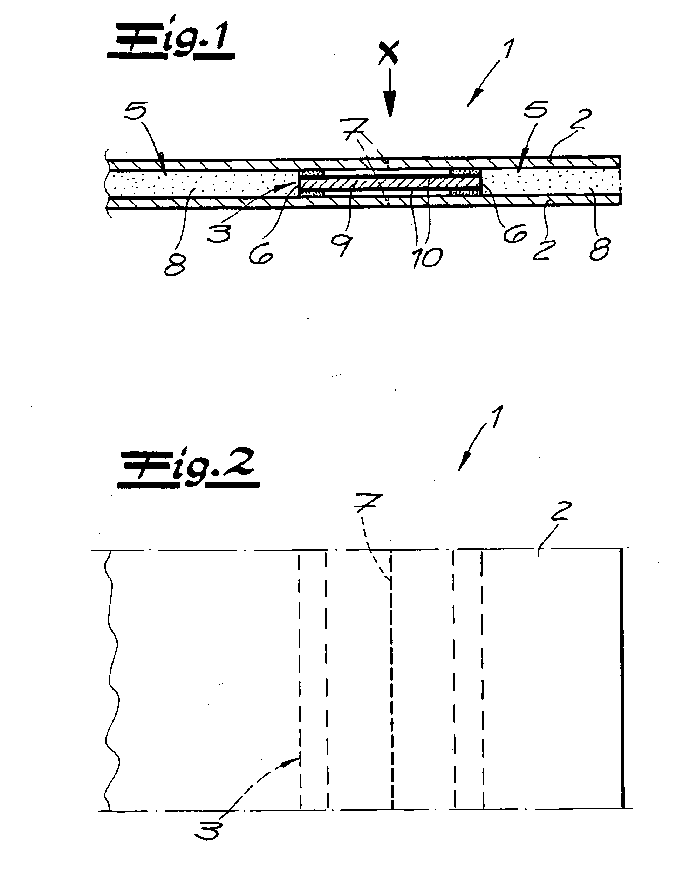

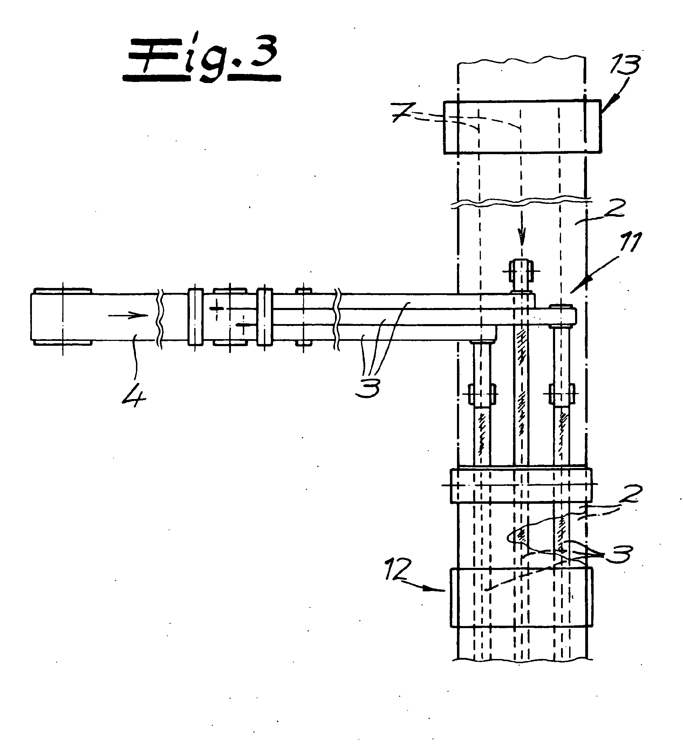

In Fig. 1 ist eine Verbundstoffbahn dargestellt, aus der einzelne Windelverschlusselemente 1 hergestellt werden können. Die Verbundstoffbahn weist zwei außenliegende Vliesstoffbahnen 2 und dazwischen abschnittsweise angeordnete Folienstreifen 3 einer elastischen Folie 4 (s. Fig. 3) auf. Die Vliesstoffbahnen 2 sind in den Bereichen 5 zwischen den Folienstreifen 3 miteinander verbunden. Die Folienstreifen 3 sind lediglich an jeweils beiden Längsrändern 6 sowohl ober- als auch unterseitig mit den Innenflächen der Vliesstoffbahnen 2 verbunden. Die Vliesstoffbahnen 2 weisen in den die Folienstreifen 3 überdeckenden Bereichen in Bahnrichtung verlaufende Schwächungen 7 auf, die im Ausführungsbeispiels als Perforationen ausgebildet sind. Pro Folienstreifen 3 besitzen die Vliesstoffbahnen 2 hierbei jeweils eine Perforation 7, welche jeweils wiederum symmetrisch zum Folienstreifen 3 verlaufen. Die Perforationen 7 erstrecken sich über die gesamte Länge der Folienstreifen 3. In den Bereichen 5 zwischen den Folienstreifen 3 sind die Vliesstoffbahnen 2 vollflächig miteinander verklebt, während die Randbereiche der Folienstreifen 3 vollflächig mit den Innenflächen der Vliesstoffbahnen 2 verklebt sind. Die Verklebung kann beispielsweise mittels eines Hotmeltsklebers 8 im Rahmen eines Kaschiervorganges erfolgen. Der Fig. 1 ist ferner zu entnehmen, dass die Folienstreifen 3 jeweils aus einer Elastomerschicht 9 und zumindest einer auf die Elastomerschicht 9 aufgebrachten Außenschicht 10 aus Vliesstoff bestehen. Im Ausführungsbeispiel ist die Elastomerschicht 9 klebrig und daher beidseitig mit dünnen Außenschichten 10 aus Vliesstoff versehen. Diese Außenschichten 10 weisen zweckmäßig ein Flächengewicht zwischen 5 g/m² und 15 g/m² auf, wohingegen die Vliesstoffbahnen 2 jeweils ein deutlich höheres Flächengewicht aufweisen.

In Fig. 3 ist ein erfindungsgemäßes Verfahren zur Herstellung einer Verbundstoffbahn dargestellt. Zunächst werden aus einer elastischen Folie 4 mittels eines Trennvorganges Folienstreifen 3 erzeugt, welche über Umlenkeinrichtungen 11 geführt und als parallel zueinander beabstandete Streifen einer Kaschiereinrichtung 12 zugeführt werden, in der die Folienstreifen 3 abschnittsweise zwischen Vliesstoffbahnen 2 angeordnet und einkaschiert werden. Mittels eines vollflächigen Klebstoffauftrages in den Bereichen 5 zwischen den Folienstreifen 3 werden die Vliesstoffbahnen 2 miteinander verbunden. Die Folienstreifen 3 werden durch einen entsprechenden Klebstoffauftrag auf der Innenfläche der beiden Verbundstoffbahnen 2 lediglich an jeweils beiden Längsrändern 6 sowohl ober- als auch unterseitig mit den Innenflächen der Vliesstoffbahnen 2 verbunden. Sowohl die Verbindung der beiden Vliesstoffbahnen 2 als auch die Verbindung der Folienstreifen 3 mit den beiden Vliesstoffbahnen 2 erfolgt in der Kaschiereinrichtung. Zuvor werden die Vliesstoffbahnen 2 in den die Folienstreifen 3 überdeckenden Bereichen symmetrisch zum jeweiligen Folienstreifen 3 in Bahnrichtung perforiert. Hierzu durchlaufen die Vliesstoffbahnen 2 eine entsprechende Perforiereinrichtung 13. Die hergestellte Verbundstoffbahn kann durch entsprechende Schneidvorgänge zu elastischen Windelverschlüssen 1 weiterverarbeitet werden, welche jeweils lediglich einen Folienstreifen 3 aufweisen.

Die miteinander verbundenen Vliesstoffbahnen 2 bilden in den Bereichen 5 zwischen den Folienstreifen 3 nicht elastische Bereiche, während die jeweils einen Folienstreifen 3 enthaltenen Abschnitte einen freilegbaren elastischen Bereich bilden. Die Vliesstoffbahnen 2 und die Außenschichten 10 der Folienstreifen 3 bestehen zweckmäßig aus denselben Rohstoffen, beispielsweise Polypropylenfasern. Hinsichtlich der Faserstruktur können sich die Außenschichten 10 der Folienstreifen 3 von den Vliesstoffbahnen 2 unterscheiden. Für die Außenschichten 10 der Folienstreifen 3 wird zweckmäßigerweise eine dichte Faserstruktur gewählt, welche verhindert, dass die zu einer Rolle aufgewickelten Lagen verblocken. Um die gewünschte textile Oberfläche zu gewährleisten, reicht jedoch eine vergleichsweise dünne Außenschichtdicke aus. Die außenseitig aufkaschierten Vliesstoffbahnen 2 weisen hingegen zweckmäßigerweise eine lockere, voluminöse Faserstruktur auf, um eine angenehme Textiloberflächenstruktur zu erzeugen, sind aber deutlich dicker als die Außenschichten 10.

## Patentansprüche

1. Verbundstoffbahn mit
zwei außenliegenden Vliesstoffbahnen (2) und
dazwischen abschnittsweise angeordneten Folienstreifen (3) einer elastischen Folie (4),
wobei die Vliesstoffbahnen (2) in den Bereichen (5) zwischen den Folienstreifen (3) miteinander verbunden sind, **dadurch gekennzeichnet, dass** die Folienstreifen (3) lediglich an jeweils beiden Längsrändern (6) sowohl oberals auch unterseitig mit den Innenflächen der Vliesstoffbahnen (2) verbunden sind und dass die Vliesstoffbahnen (2) in dem die Folienstreifen (3) überdeckenden Bereichen in Bahnrichtung verlaufende Schwächungen (7) aufweisen.

2. Verbundstoffbahn nach Anspruch 1, **dadurch gekennzeichnet, dass** die Vliesstoffbahnen (2) pro Folienstreifen (3) jeweils eine Schwächung (7) aufweisen und dass die Schwächungen (7) jeweils symmetrisch zum Folienstreifen (3) verlaufen.

3. Verbundstoffbahn nach Anspruch 1 oder 2, **dadurch gekennzeichnet, dass** sich die Schwächungen (7) jeweils über die gesamte Länge der Folienstreifen (3) erstrecken.

4. Verbundstoffbahn nach einem der Ansprüche 1 bis 3, **dadurch gekennzeichnet, dass** die Vliesstoffbahnen (2) in den Bereichen zwischen den Folienstreifen (3) miteinander verklebt sind.

5. Verbundstoffbahn nach einem der Ansprüche 1 bis 4, **dadurch gekennzeichnet, dass** die Randbereiche der Folienstreifen (3) mit den Innenflächen der Vliesstoffbahnen (2) verklebt sind.

6. Verbundstoffbahn nach einem der Ansprüche 1 bis 5, **dadurch gekennzeichnet, dass** die Folienstreifen (3) jeweils aus einer Elastomerschicht (9) und zumindest einer auf die Elastomerschicht (9) aufgebrachten Außenschicht (10) aus Vliesstoff bestehen.

7. Verbundstoffbahn nach Anspruch 6, **dadurch gekennzeichnet, dass** die Elastomerschicht (9) beidseitig mit Außenschichten (10) aus Vliesstoff versehen ist.

8. Verfahren zur Herstellung einer Verbundstoffbahn,
wobei aus einer elastischen Folie mittels eines Trennvorganges Folienstreifen erzeugen werden,
wobei die Folienstreifen danach abschnittsweise zwischen Vliesstoffbahnen angeordnet werden
und wobei danach die Vliesstoffbahnen in den Bereichen zwischen den Folienstreifen miteinander verbunden werden,
**dadurch gekennzeichnet, dass** die Folienstreifen lediglich an jeweils an beiden Längsrändern sowohl ober- als auch unterseitig mit den Innenflächen der Vliesstoffbahnen verbunden werden und dass die Vliesstoffbahnen in den die Folienstreifen überdeckenden Bereichen in Bahnrichtung geschwächt werden.

9. Verfahren nach Anspruch 8, **dadurch gekennzeichnet, dass** die Vliesstoffbahnen pro Folienstreifen jeweils einmal symmetrisch zum Folienstreifen geschwächt werden.

10. Verfahren nach Anspruch 8 oder 9, **dadurch gekennzeichnet, dass** die Vliesstoffbahnen in den Bereichen zwischen den Folienstreifen miteinander verklebt werden und die Randbereiche der Folienstreifen mit den Innenflächen der Vliesstoffbahnen verklebt werden.

## Claims

1. A composite material web comprising
two outer nonwoven fabric webs (2) and
film strips (3) of an elastic film (4) disposed therebetween in sections,
wherein said nonwoven fabric webs (2) are connected to one another between the film strips (3)**characterized in that** said film strips (3) are only connected to the inner surfaces of said nonwoven fabric webs (2) at respectively both longitudinal edges (6) both on the top side and on the bottom side and that said nonwoven fabric webs (2) have weakenings (7) running in the web direction in regions covering the film strips (3).

2. The composite material web according to claim 1, **characterized in that** the nonwoven fabric webs (2) have respectively one weakening (7) per film strip (3) and that the weakenings (7) each run symmetrically to the respective film strip (3).

3. The composite material web according to claim 1 or 2, wherein the weakenings (7) each extend over the entire length of the film strips (3).

4. The composite material web according to any one of claims 1 to 3, **characterized in that** the nonwoven fabric webs (2) are glued to one another in the regions between the film strips (3).

5. The composite material web according to any one of claims 1 to 4, **characterized in that** the edge regions of the film strips (3) are glued to the inner surfaces of the nonwoven fabric webs (2).

6. The composite material web according to any one of claims 1 to 5, **characterized in that** the film strips (3) each consist of an elastomer layer (9) and at least one outer layer (10) of nonwoven fabric applied to the elastomer layer (9).

7. The composite material web according to claim 6, **characterized in that** the elastomer layer (9) is provided on both sides with outer layers (10) of nonwoven fabric.

8. A method for producing a composite material
wherein film strips are produced from an elastic film by means of a cutting process,
wherein the film strips are then arranged between nonwoven fabric webs in sections
and wherein the nonwoven fabric webs are then connected to one another in the regions between the film strips,
**characterized in that** the film strips are only connected to one another at respectively both longitudinal edges both on the top side and on the bottom side and that said nonwoven fabric webs are weakened in the web direction in regions covering the film strips.

9. The method according to claim 8, **characterized in that** the nonwoven fabric webs are each weakened once per film strip symmetrically to the film strip.

10. The method according to claim 8 or 9, **characterized in that** the nonwoven fabric webs are glued to one another in the regions between the film strips, and the edge regions of the film strips are glued to the inner surfaces of the nonwoven fabric webs.

## Revendications

1. Bande de matériau composite comportant
deux bandes de non-tissé extérieures (2) et
des bandes de film (3) en film élastique (4) disposées entre elles par sections,
les bandes de non-tissé (2) étant reliées entre elles dans les zones (5) situées entre les bandes de film (3), **caractérisée en ce que** les bandes de film (3) sont reliées simplement au niveau respectivement des deux bords longitudinaux aussi bien en haut qu'en bas aux surfaces intérieures des bandes de non-tissé (2) et que les bandes de non-tissé (2) présentent des parties moins résistantes (7) s'étendant dans le sens de la bande dans les zones recouvrant les bandes de film (3).

2. Bande de matériau composite selon la revendication 1, **caractérisée en ce que** les bandes de non-tissé (2) présentent respectivement par bande de film une partie moins résistante (7) et que les parties moins résistantes (7) s'étendent respectivement en direction de la bande de film (3).

3. Bande de matériau composite selon la revendication 1 ou 2, **caractérisée en ce que** les parties moins résistantes (7) s'étendent respectivement sur toute la longueur des bandes de film (3).

4. Bande de matériau composite selon une des revendications 1 à 3, **caractérisée en ce que** les bandes de non-tissé (2) sont collées ensemble dans les zones situées entre les bandes de film (3).

5. Bande de matériau composite selon une des revendications 1 à 4, **caractérisée en ce que** les parties périphériques des bandes de film (3) sont collées aux surfaces intérieures des bandes de non-tissé (2).

6. Bande de matériau composite selon une des revendications 1 à 5, **caractérisée en ce que** les bandes de film (3) sont composées respectivement d'une couche d'élastomère (9) et d'au moins une couche extérieure (10) en non-tissé appliquée sur la couche d'élastomère (9).

7. Bande de matériau composite selon la revendication 6, **caractérisée en ce que** la couche d'élastomère (9) est pourvue bilatéralement de couches extérieurs (10) en non-tissé.

8. Procédé de fabrication d'une bande de matériau composite, dans lequel
des bandes de film sont fabriquées à partir d'un film élastique au moyen d'une opération de sectionnement,
les bandes de film sont ensuite disposées par sections entre les bandes de non-tissé
et les bandes de non-tissé sont ensuite reliées entre elles dans les zones situées entre les bandes de film,
**caractérisé en ce que** les bandes de film sont reliées simplement aux surfaces intérieures des bandes de non-tissé respectivement au niveau des deux bords longitudinaux aussi bien en haut qu'en bas et que les bandes de non-tissé sont rendues moins résistantes dans les zones recouvrant les bandes de film.

9. Procédé selon la revendication 8, **caractérisé en ce que** les bandes de non-tissé, pour chaque bande de film, sont rendues moins résistantes respectivement une fois symétriquement par rapport à la bande de film.

10. Procédé selon la revendication 8 ou 9, **caractérisé en ce que** les bandes de non-tissé sont collées ensemble dans les zones situées entre les bandes de film et que les parties périphériques des bandes de film sont collées aux surfaces intérieures des bandes de non-tissé.
